# FASCICULE DE BREVET EUROPEEN

(11) **EP 2 893 010 B1**
(45) Date de publication et mention de la délivrance du brevet: **05.04.2017**
(21) Numéro de dépôt: 13774720.0
(22) Date de dépôt: 04.09.2013
(51) Int. Cl.: C12N 9/42, C12P 7/10, C12P 19/02, C12P 19/14, C12P 7/14

(54) **POLYPEPTIDE À ACTIVITÉ BETA-GLUCOSIDASE RENFORCÉE À BASSE TEMPÉRATURE**
POLYPEPTID MIT VERSTÄRKTER BETA-GLUCOSIDASE-AKTIVITÄT BEI NIEDRIGER TEMPERATUR
POLYPEPTIDE WITH REINFORCED BETA-GLUCOSIDASE ACTIVITY AT LOW TEMPERATURE

(30) Priorité: 05.09.2012 FR 1258260
(43) Date de publication de la demande: 15.07.2015
(73) Titulaire: IFP Énergies nouvelles, 92852 Rueil-Malmaison Cedex (FR); Proteus, 91160 Longjumeau (FR); Centre National de la Recherche Scientifique (C.N.R.S.), 75794 Paris Cedex 16 (FR)
(72) Inventeur: MARGEOT, Antoine, 75018 Paris (FR); MATHIS, Hugues, 77600 Bussy Saint Georges (FR); AYRINHAC, Céline, 30350 Domessargues (FR); ULLMANN, Christophe, 30000 Nimes (FR); PERSILLON, Cécile, 30000 Nimes (FR); FORT, Sébastien, 38410 Uriage (FR); ARMAND, Sylvie, 38100 Grenoble (FR); PETIT, Maud, 59700 Marcq en Baroeul (FR)
(74) Mandataire: Cabinet Plasseraud
(86) Numéro de dépôt international: PCT/FR2013/052036
(87) Numéro de publication internationale: WO 2014/037667

(56) Documents cités:
- WO-A1-92/10581
- WO-A1-2010/029259
- WO-A2-2010/148148
- US-A1- 2010 304 438
- DASHTBAN MEHDI ET AL: "Fungal Bioconversion of Lignocellulosic Residues; Opportunities & Perspectives", INTERNATIONAL JOURNAL OF BIOLOGICAL SCIENCES, IVYSPRING INTERNATIONAL PUBLISHER, AU, vol. 5, no. 6, 4 septembre 2009 (2009-09-04), pages 578-595, XP002570268, ISSN: 1449-2288
- CÉLINE AYRINHAC ET AL: "Improved Saccharification of Wheat Straw for Biofuel Production Using an Engineered Secretome of Trichoderma reesei", ORGANIC PROCESS RESEARCH & DEVELOPMENT, vol. 15, no. 1, 21 janvier 2011 (2011-01-21), pages 275-278, XP055086689, ISSN: 1083-6160, DOI: 10.1021/op100218a

## Description

La possibilité de produire de l'éthanol à partir de la cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La cellulose est un polymère constitué de molécules de glucose reliées par des liaisons beta 1-4, qui sont très résistantes à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients tels que la mauvaise récupération de l'acide lors de l'utilisation d'acides concentrés et la faible production de glucose dans le cadre de l'utilisation d'acides dilués nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes. A ce titre, beaucoup de microorganismes comportent des enzymes qui hydrolysent la cellulose, tels que les champignons *Trichoderma, Aspergillus, Humicola, Fusarium* ainsi que les bactéries telles que *Thermomonospora, Bacillus, Cellulomonas* et *Streptomyces.* Les enzymes sécrétées par ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les beta-glucosidases qui vont hydrolyser ce cellobiose en glucose. Ces dernières constituent l'étape limitante du procédé de conversion de la cellulose. En effet, la difficulté première du procédé réside dans la conversion du cellobiose en glucose, car tout cellobiose non hydrolysé à la fin du procédé représente une perte de rendement lors de la production de biocarburant.

Cette accumulation de cellobiose est un problème majeur dans l'hydrolyse enzymatique, étant donné que plusieurs microorganismes producteurs de cellulases, dont *Trichoderma,* produisent très peu de beta-glucosidase. En fait, moins de 1% des protéines totales sécrétées par des souches de *Trichoderma* industrielles sont de type beta-glucosidase. Cette faible quantité de beta-glucosidase résulte donc en une faible capacité à hydrolyser le cellobiose en glucose ; de là, son accumulation dans le système. Or une forte concentration de cellobiose inhibe l'activité des autres cellulases et notamment les exoglucanases pour qui le cellobiose est le produit final de la réaction. Pour pallier ces inconvénients, les inventeurs ont développé dans leur demande de brevet WO2010/029259 des gènes de beta-glucosidase permettant l'obtention d'enzymes à activité spécifique augmentée, ce qui améliore sensiblement le procédé de conversion de biomasse lignocellulosique en biocarburant.

L'hydrolyse et la fermentation peuvent être réalisées suivant différents schémas. Le plus courant consiste en une hydrolyse et une fermentation séparées (SHF - Separate Hydrolysis and Fermentation). Cette méthode permet d'optimiser chaque étape par le maintien des conditions optimales de réaction. Cette fermentation s'effectue de manière extemporanée, à une température comprise entre environ 28°C et environ 30°C, tandis que l'hydrolyse a lieu généralement à une température d'au moins 45°C. Cependant, en SHF, les sucres libérés en fin de réaction sont à très forte concentration et entraînent une inhibition des enzymes, ralentissant l'efficacité du procédé.

Pour éviter ces inconvénients, un autre type de procédé (SSF- Simultaneous Saccharification and Fermentation) peut être envisagé. En SSF, les deux étapes (hydrolyse et fermentation des hexoses) ont lieu de manière simultanée empêchant l'accumulation des sucres à des concentrations inhibitrices pour les enzymes. Les coûts d'investissement sont également réduits suite à l'utilisation d'un seul réacteur. Le taux d'hydrolyse est plus élevé suite à l'absence d'inhibition car les sucres libérés sont utilisés immédiatement pour la fermentation en éthanol.

Dans cette méthode, la température du réacteur constitue nécessairement un compromis entre les températures optimales d'hydrolyse et de fermentation, typiquement entre environ 30°C et environ 35°C. Cependant, à une telle température, l'activité des enzymes cellulolytiques, dont la beta-glucosidase, est diminuée de 30% environ.

Il existe donc un besoin pour des enzymes susceptibles de maintenir une activité beta-glucosidase efficace aux températures optimales d'hydrolyse et de fermentation d'un procédé SSF, en particulier à une température comprise entre environ 30°C et environ 35°C.

Les inventeurs ont développé un polypeptide ayant une activité beta-glucosidase améliorée à une température comprise entre environ 30°C et environ 35°C, notamment par rapport à l'activité beta-glucosidase de la protéine sauvage BGL1 de séquence SEQ ID N°3. BGL1 correspond à la beta-glucosidase de *Trichoderma reesei.*

Les inventeurs ont précédemment identifié plusieurs clones ayant une activité spécifique beta-glucosidase améliorée par rapport à l'activité beta-glucosidase de la protéine sauvage BGL1. De tels résultats sont présentés dans leur demande de brevet WO 2010/029259. Plus spécifiquement, ils ont mis en évidence un clone particulier codant pour un polypeptide de séquence SEQ ID N°5 (dénommé 100B11) dont l'expression dans *Trichoderma reesei* sous contrôle d'un promoteur fort entraine un facteur d'augmentation de l'activité beta-glucosidase de 26,2 (tableau 6 de la demande de brevet WO 2010/029259) du cocktail enzymatique produit par rapport à celui produit par une souche n'exprimant pas cette enzyme.

Ils ont maintenant mis en évidence, de manière surprenante et inattendue, un nouveau clone, qui code pour une enzyme présentant une activité améliorée par rapport au clone 100B11 précédemment identifié, et ceci à une température comprise entre environ 30°C et environ 35°C.

L'invention concerne donc un polypeptide ayant une activité beta-glucosidase de séquence d'acides aminés SEQ ID N°1.

La séquence d'acides aminés du polypeptide de l'invention est comme suit:

Ce polypeptide est codé par la séquence d'acides nucléiques SEQ ID N°2.

Préférentiellement, ledit polypeptide de séquence d'acides aminés SEQ ID N°1 a une activité beta-glucosidase à une température comprise entre environ 30°C et environ 35°C améliorée, notamment par rapport à l'activité beta-glucosidase de la protéine sauvage BGL1 de séquence SEQ ID N°3 à ces mêmes températures. La protéine BGL1 est codée par la séquence d'acides nucléiques SEQ ID N°4.

Plus préférentiellement, ledit polypeptide de séquence d'acides aminés SEQ ID N°1 a une activité beta-glucosidase à une température comprise entre environ 30°C et environ 35°C améliorée par rapport à l'activité beta-glucosidase du polypeptide 100B11 de séquence d'acides aminés SEQ ID N°5 à ces mêmes températures. Le polypeptide 100B11 est codé par la séquence d'acides nucléiques SEQ ID N°6.

De plus, le polypeptide selon l'invention présente l'avantage d'être moins sensible à l'inhibition par le glucose et conserve de ce fait une meilleure activité beta-glucosidase en présence d'une forte concentration de glucose.

Dans un mode de réalisation, le polypeptide tel que décrit précédemment a une activité beta-glucosidase déterminée en présence de glucose qui est améliorée par rapport à l'activité beta-glucosidase de la protéine sauvage BGL1 (SEQ ID N°3) déterminée en absence de glucose.

Dans un mode de réalisation préféré, le polypeptide de l'invention a une activité beta-glucosidase améliorée d'au moins 10%, préférentiellement d'au moins 20%, préférentiellement d'au moins 30%, encore plus préférentiellement d'au moins 40% à une température comprise entre environ 30°C et environ 35°C par rapport à l'activité beta-glucosidase du polypeptide 100B11 de séquence d'acides aminés SEQ ID N°5.

L'homme du métier pourra par exemple déterminer l'augmentation ou autrement dit l'amélioration de l'activité enzymatique d'un polypeptide selon l'invention par un test d'activité enzymatique à l'aide du substrat para-nitrophényl beta-D-glucopyranoside (pNPG). La quantité de para-nitrophénol obtenue après action de la beta-glucosidase pourra par exemple être déterminée par lecture de la densité optique à 414 nm.

Un exemple de protocole, que l'homme du métier pourra utiliser pour déterminer si un polypeptide selon l'invention présente une activité enzymatique améliorée par rapport à celle de la protéine sauvage BGL1, est le suivant :
- préparation d'une culture stock de *E. coli* exprimant un polypeptide selon l'invention pendant toute la nuit à 37°C ;
- ensemencement d'un milieu de culture LB avec 1% de culture stock pendant 24h à 20°C;
- centrifugation pendant 2 minutes à 13000 rpm ;
- resuspension des culots cellulaires avec du tampon succinate 100 mM à pH 5 (DO₆₀₀ finale = 100);
- incubation de 50 µL de cellules avec 100 µL de tampon succinate 100 mM à pH 5 contenant 15 mM de para-nitrophényl beta-D-glucopyranoside (pNPG) pendant 1h30 à 50°C, suivi de 5 minutes sur la glace ;
- ajout de 150 µL de Na₂CO₃ 0,2 M ;
- centrifugation pendant 2 minutes à 13000 rpm ;
- lecture de la densité optique à 414 nm sur 150 µL de surnageant.

De plus, l'homme du métier pourra utiliser le protocole décrit ci-dessus en incubant les 50 µL de cellules avec 100 µL de tampon succinate 100 mM à pH 5 contenant 15 mM de pNPG et 60 g/L de glucose pendant 1h30 à 50°C, pour déterminer si un polypeptide selon l'invention est moins sensible à l'inhibition par le glucose que la protéine sauvage BGL1.

Ces protocoles sont aisément adaptables pour mesurer l'amélioration de l'activité beta-glucosidase dans des conditions de température comprises entre environ 30°C et environ 35°C, notamment par rapport au polypeptide 100B11 de séquence d'acides aminés SEQ ID N°5.

L'invention concerne également un acide nucléique codant pour le polypeptide de séquence d'acides aminé SEQ ID N°1. Préférentiellement, ledit acide nucléique comprend la séquence d'acides nucléiques SEQ ID N°2.

L'invention porte également sur un vecteur comprenant un acide nucléique tel que décrit précédemment.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. Des exemples de vecteurs sont les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus.

Selon l'invention, l'acide nucléique tel que décrit précédemment pourra être lié opérationnellement à un promoteur, un terminateur ou toute autre séquence nécessaire à son expression dans la cellule hôte.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée capable de produire le polypeptide de l'invention tel que décrit précédemment, ou comprenant un acide nucléique codant pour ledit polypeptide de l'invention.

L'homme du métier pourra introduire au moins le polypeptide, l'acide nucléique ou le vecteur tels que décrit précédemment dans la cellule hôte par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte et par des méthodes conventionnelles, plusieurs copies d'un acide nucléique codant un polypeptide ayant une activité beta-glucosidase améliorée selon l'invention.

Selon un mode de réalisation, la cellule hôte isolée telle que décrite précédemment est choisie parmi *Trichoderma, Aspergillus, Neurospora, Humicola, Myceliophthora, Chrysosporium, Penicillium, Fusarium, Thermomonospora, Bacillus, Pseudomonas, Escherichia, Clostridium, Cellulomonas, Streptomyces, Yarrowia, Pichia* et *Saccharomyces.*

Selon un mode de réalisation préféré, la cellule hôte isolée telle que décrite précédemment est choisie parmi *Trichoderma reesei, Trichoderma viridae, Trichoderma koningü, Aspergillus niger, Aspergillus nidulans, Myceliophthora thermopila, Chrysosporium lucknowense, Aspergillus wentü, Aspergillus oryzae, Aspergillus phoenicis, Neurospora crassa, Humicola grisae, Penicillium pinophilum, Penicillium oxalicum, Escherichia coli, Clostridium acetobutylicum, Clostridium saccharolyticum, Clostridium benjerinckü, Clostridium butylicum, Pichia pastoris, Yarrowia lipolityca, Saccharomyces cerevisiae et* leurs mélanges.

Selon un mode de réalisation préféré, la cellule hôte isolée telle que décrite précédemment est choisie parmi *Trichoderma reesei* et *Saccharomyces cerevisiae.*

L'invention porte également sur l'utilisation du polypeptide tel que décrit précédemment ou l'une quelconque des cellules décrites précédemment pour l'hydrolyse des beta-oligosaccharides.

L'invention porte également sur l'utilisation du polypeptide tel que décrit précédemment ou l'une quelconque des cellules décrites précédemment pour l'hydrolyse du cellobiose en glucose.

L'invention a également pour objet, l'utilisation du polypeptide tel que décrit précédemment ou l'une quelconque des cellules décrites précédemment pour la production de biocarburant.

Selon l'invention, le terme biocarburant peut être défini comme tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Dans un autre mode de réalisation, le produit est une molécule intéressant l'industrie chimique tels que par exemple d'autres alcools tels que le 1,2-propane diol, le 1,3-propane diol, le 1,4-butane diol, le 2,3-butane diol, des acides organiques comme l'acide acétique, propionique, acrylique, butyrique, succinique, malique, fumarique, citrique, itaconique, ou des hydroxyacides comme l'acide glycolique, hydroxypropionique, ou lactique.

Outre la production de biocarburant, le polypeptide ayant une activité beta-glucosidase améliorée à une température comprise entre 30°C et 35°C peut également être utilisé dans d'autres types d'applications en catalysant l'hydrolyse de divers substrats, permettant ainsi la libération d'une variété d'arômes. A titre d'exemple, il peut être utilisé afin de libérer des arômes de fruits en hydrolysant plusieurs glucosides présents à l'intérieur de ces fruits ou encore, il peut hydrolyser les beta-glucosides monoterphényl de raisins représentant ainsi une source importante d'arômes pour le vin. Par conséquent, le polypeptide tel que décrit précédemment peut être utilisé dans plusieurs domaines, notamment en parfumerie, en industrie alimentaire, en oenologie, etc.

Les souches de champignons filamenteux, de préférence *Trichoderma,* plus préférentiellement *T. reesei,* capables d'exprimer le polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g/L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment les endoglucanases, les exoglucanases et la beta-glucosidase selon l'invention. Dans un mode de réalisation, la solution aqueuse contenant le substrat choisi pour la production des enzymes est préparée à la concentration de 200-250 g/L ; cette solution doit contenir un substrat inducteur tel que le lactose. Cette solution aqueuse est injectée après l'épuisement du substrat carboné initial de façon à apporter une quantité optimisée, comprise entre 35 et 45 mg/g de cellules ("fed batch"). Pendant cette phase de "fed batch", la concentration résiduelle en sucre dans le milieu de culture est inférieure à 1 g/L et les enzymes agissant sur la biomasse lignocellulosique sont sécrétées par le champignon. Ces dernières peuvent être récupérées par filtration du milieu de culture.

L'invention a pour objet une composition enzymatique agissant sur la biomasse lignocellulosique, ladite composition enzymatique étant produite par des champignons filamenteux ou des levures et comprenant le polypeptide tel que décrit précédemment.

Enfin, l'invention a pour objet un procédé de production de biocarburant à partir de la biomasse comprenant les étapes suivantes :
- mise en suspension en phase aqueuse du matériau à hydrolyser ;
- hydrolyse en présence d'une composition enzymatique de la biomasse lignocellulosique telle que décrite précédemment de manière à produire un hydrolysat contenant du glucose;
- fermentation du glucose de l'hydrolysat ;
- séparation du biocarburant du moût de fermentation,
caractérisé en ce que les étapes d'hydrolyse et de fermentation se font de manière simultanée.

Un autre objet de l'invention est un procédé de production de biocarburant à partir de la biomasse, caractérisé en ce qu'il comprend les étapes suivantes :
- mise en suspension en phase aqueuse de la biomasse à hydrolyser ;
- ajout simultané d'une composition enzymatique agissant sur la biomasse lignocellulosique telle que définie précédemment et d'un organisme fermentaire et incubation;
- séparation du biocarburant du moût de fermentation.

Un autre objet de l'invention est un procédé de production de biocarburant à partir de la biomasse, caractérisé en ce qu'il comprend les étapes successives suivantes :
- mise en suspension en phase aqueuse de la biomasse à hydrolyser ;
- ajout d'un ou plusieurs organismes cellulolytiques et/ou fermentaires comme définis précédemment à une température comprise entre 30°C et 35°C de manière à produire un moût de fermentation;
- séparation du biocarburant du moût de fermentation.

Selon ce mode de réalisation, la cellulose présente dans la biomasse est convertie en glucose, et en même temps, dans le même réacteur, l'organisme fermentaire (par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, l'organisme fermentaire produit le polypeptide objet de l'invention, par sécrétion ou en surface de sa cellule, éventuellement conjointement à d'autres enzymes agissant sur la biomasse lignocellulosique, limitant ou supprimant ainsi le besoin en enzymes produites par le champignon filamenteux.

L'utilisation du polypeptide présentant une meilleure activité beta-glucosidase à une température comprise entre environ 30°C et environ 35°C selon la présente invention présente ainsi l'avantage d'obtenir un meilleur rendement de production de glucose. Ainsi la présente invention permet d'utiliser moins d'enzyme qu'auparavant, ce qui présente un avantage économique, le coût de production du biocarburant, par exemple, étant moindre.

D'autres aspects, objets, avantages et caractéristiques de l'invention, seront présentés à la lecture de la description non restrictive qui suit et qui décrit des modes de réalisation préférés de l'invention donnés par le biais d'exemples.

### EXEMPLES

### EXEMPLE 1 : 1er tour de shuffling

La séquence du gène de la beta-glucosidase de *Trichoderma reesei* (gène parental BGL1, SEQ ID N°4) a été soumise à un premier tour de shuffling selon le procédé breveté décrit dans EP 1104457B1 avec le gène de la glucosidase putative de *Chaetomium globosum* (gène A) (SEQ ID NO: 7, codé par la séquence d'acides nucléiques SEQ ID N°8) possédant 70% d'identité avec le gène parental BGL1.

### 1- Criblage à haut débit

Un test de criblage à haut débit a permis de sélectionner les meilleurs clones issus du shuffling de ces deux séquences, c'est-à-dire ceux présentant un facteur d'amélioration supérieur à 2 au niveau de l'activité beta-glucosidase lorsque comparés avec le gène parental BGL1 de *T. reesei.*

Le test de criblage de la banque du premier tour de shuffling a été réalisé selon les étapes suivantes :
- isolement sur gélose des différentes colonies de *E.coli* exprimant les variants de shuffling de l'enzyme recombinante selon l'invention et mise en pré-cultures en milieu LB desdites colonies pendant la nuit à 37°C;
- inoculation d'un milieu LB à 3% avec la pré-culture puis incubation 4h à 37°C ;
- induction de l'expression des variants par addition d'isopropyl-beta-thio-galactoside (IPTG) 100 µM puis incubation à 20°C pendant la nuit ;
- centrifugation pendant 2 minutes à 13000 rpm ;
- re-suspension des culots cellulaires dans 100 µL de tampon succinate 0,1M contenant 2,2 mM de para-nitrophényl beta-D-glucopyranoside (pNPG) ;
- incubation 3h à température ambiante ;
- lecture de la densité optique à 414 nm après alcalinisation.

Dans ces conditions de criblage, plusieurs clones présentant une amélioration de l'activité beta-glucosidase par rapport à l'enzyme de référence BGLI ont été identifiés.

### 2-Détermination de l'amélioration de l'activité beta-slucosidase.

### 2-1/ Sur le substrat pNPG

Afin de déterminer le kcat relatif des variants sélectionnés au premier tour de shuffling, on procède de la façon suivante :
- formation d'une culture stock de *E. coli* exprimant une enzyme recombinante selon l'invention pendant toute la nuit à 37°C ;
- ensemencement d'un milieu de culture LB avec 1% de culture stock pendant 24h à 20°C avec induction à l'IPTG (250 µM);
- centrifugation pendant 2 minutes à 13000 rpm ;
- resuspension des culots cellulaires avec du tampon succinate 100 mM à pH 5 (DO₆₀₀ finale = 100);
- incubation de 50 µL de cellules avec 100 µL de tampon succinate 100 mM à pH 5 contenant 15 mM de para-nitrophényl beta-D-glucopyranoside (pNPG) pendant 1h30 à 50°C suivi de 5 minutes sur la glace ;
- ajout de 150 µL de Na₂CO₃ 0,2 M ;
- centrifugation pendant 2 minutes à 13000 rpm ;
- lecture de la densité optique à 414 nm sur 150 µL de surnageant.

Le tableau 2 présente les valeurs des kcat ainsi que les facteurs d'améliorations obtenus pour trois clones identifiés précédemment (dénommés 10H7, 59B8 et 164A2) dans ces conditions expérimentales.

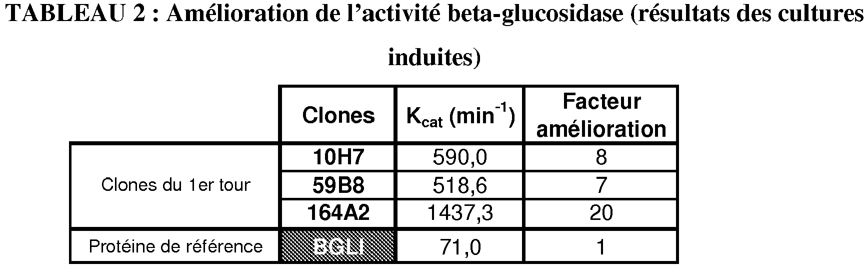

Les résultats montrent des améliorations d'activités enzymatiques très importantes par rapport à l'enzyme sauvage (BGLI) pour les 3 clones 10H7, 59B8 et 164A2.

### 2-2/ Sur le cellobiose

L'amélioration d'activité des clones 10H7, 59B8 et 164A2 a ensuite été confirmée sur un second substrat : le cellobiose.

Ce test a été effectué sur des cultures de *E. coli* exprimant une enzyme recombinante selon l'invention. Les étapes du test sont les suivantes :
- ensemencer un milieu de culture LB avec 1% de culture stock induites avec de l'IPTG puis incubation toute la nuit à 37°C.
- cultiver lesdites cellules à 37°C jusqu'à l'obtention d'une densité optique à 600 nm de 0,4.
- induire lesdites cellules avec de l'IPTG 250 µM à 20°C pendant 20 heures.
- laver les culots cellulaires trois fois dans un tampon succinate 100 mM pH 5 pour éliminer le glucose du milieu de culture.
- préparer un mix réactionnel (MR1) constitué de 10 µL desdites cellules et de 190 µL de cellobiose à 263,2 mM (250 mM finale) pendant 12 heures à 50°C en microplaque
- incuber pendant 12 heures à 50°C en microplaque.

### Révélation :

- Préparer un mix réactionnel (MR2) constitué de:
   - 10 µL de MR1
   - 90 µL de tampon succinate 100 mM à pH 5
   - 5 µL de glucose oxydase à 44 U/mL
- Incuber pendant 1h à température ambiante
- Mélanger et incuber pendant 30 min à température ambiante
   - 10 µL de MR2
   - 2 µL de horse radish peroxydase à 10 U/mL
   - 5 µL d'ABTS 100 mM
   - 83 µL de tampon phosphate 50 mM pH 7,4
- Lire les densités optiques à 420 nm.

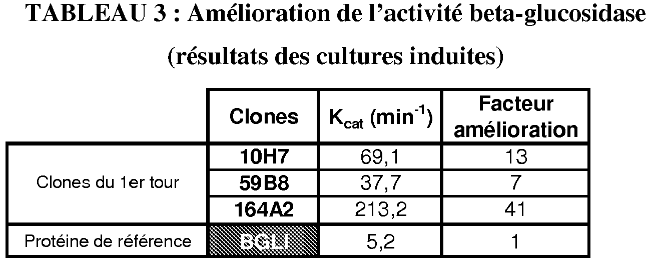

De même, les résultats montrent des améliorations d'activités enzymatiques très importantes par rapport à l'enzyme sauvage (BGL1) pour les clones 10H7, 59B8 et 164A2 lorsque le cellobiose est utilisé comme substrat.

### EXEMPLE 2 : 2eme tour de shuffling

Les séquences des gènes améliorés obtenu au premier tour de shuffling ont par la suite été soumises à un deuxième tour de shuffling (toujours selon le procédé breveté décrit dans EP1104457B1). Afin d'augmenter la diversité génétique, au moins un gène codant pour une beta-glucosidase ayant 70% d'identité avec l'enzyme sauvage BGL1 a été ajouté.

Plus précisément, le gène de la glucosidase putative de *Neurospora crassa* (gène C) (SEQ ID N°9 codé par la séquence d'acides nucléiques SEQ ID N°10) a été utilisé.

### 1- Criblage à haut débit

Un test de criblage à haut débit tel que décrit précédemment (à l'exception de l'étape d'induction à l'IPTG, puisque l'amélioration apportée au premier tour de shuffling permet une détection de l'activité beta-glucosidase basée uniquement sur la fuite du promoteur) a été effectué sur les clones obtenus à la suite de ce second tour de shuffling, afin de sélectionner les meilleurs clones, c'est-à-dire ceux présentant un facteur d'amélioration supérieur à 2 au niveau de l'activité beta-glucosidase lorsque comparés avec le clone 164A2.

Dans ces conditions de criblage, une amélioration de l'activité beta-glucosidase par rapport à l'enzyme de référence (164A2) a été trouvée dans plusieurs clones, dont notamment les clones 100B11 (SEQ ID NO : 5 codé par la séquence d'acides nucléiques SEQ ID N°6), 115E1 (SEQ ID NO : 11 codé par la séquence d'acides nucléiques SEQ ID N°12).

### 2-Détermination de l'amélioration de l'activité beta-slucosidase.

### 2-1/ Sur le pNPG

Afin de déterminer le kcat relatif, les activités des clones 100B11 et 115E1 ont été mesurées par le test d'activité tel que décrit précédemment.

Le tableau 4 présente les valeurs des kcat ainsi que les facteurs d'améliorations obtenus pour les clones 100B11 et 115E1 dans ces conditions expérimentales.

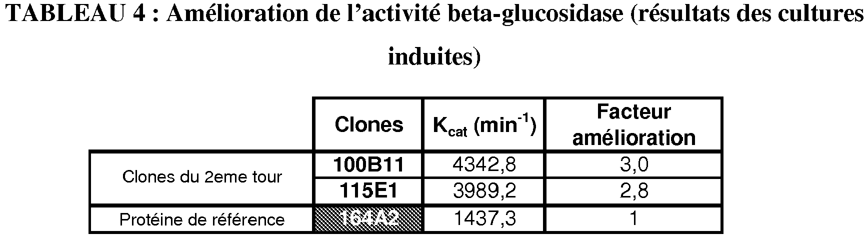

Les résultats montrent des améliorations d'activités enzymatiques très importantes par rapport à l'enzyme de référence (164A2) et à (BGLI) (X60) pour les clones 100B11 et 115E1.

### 2-2/ Sur le cellobiose

L'amélioration d'activité des clones 100B11 et 115E1 a ensuite été confirmée sur un second substrat : le cellobiose.

Afin de déterminer le kcat relatif, les activités des clones 100B11 et 115E1 ont été mesurées par le test d'activité à 50°C tel que décrit précédemment en utilisant le cellobiose comme substrat comme décrit au point 2-2 de l'exemple 1.

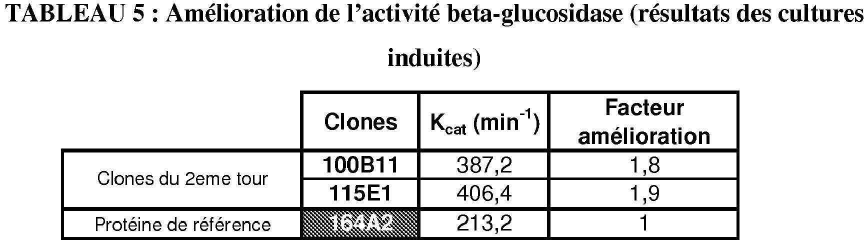

De même, les résultats montrent des améliorations d'activités enzymatiques importantes par rapport à l'enzyme de référence (164A2) pour les clones 100B11 et 115E1 lorsque le cellobiose est utilisé comme substrat.

### EXEMPLE 3 : 3ème tour de shuffling

Les séquences de 14 gènes améliorés (138E12, 134G2, 100B11, 115E1, 99G11, 127B12, 91F6, 135F9, 116D9, 212D11, 210A6, 124F5, 129D2 et 141F7) obtenus au deuxième tour de shuffling ont par la suite été soumises à un troisième tour de shuffling (toujours selon le procédé breveté décrit dans EP1104457B1). Afin d'augmenter la diversité génétique, au moins un gène codant pour une beta-glucosidase ayant 70% d'identité avec ces gènes a été ajouté. Dans cet exemple précis, le gène de la beta-glucosidase putative de *Neurospora crassa* (gène C) (SEQ ID N°9 codé par la séquence d'acides nucléiques SEQ ID N°10) et le gène de la beta-glucosidase putative de *Chaetomium globosum* (gène A) (SEQ ID N°7 codé par la séquence d'acides nucléiques SEQ ID N°8) ont été utilisés.

### 1- Criblage à haut débit

Un test de criblage à haut débit tel que décrit précédemment (à l'exception de l'étape d'induction à l'IPTG, puisque l'amélioration apportée au premier tour de shuffling permet une détection de l'activité beta-glucosidase basée uniquement sur la fuite du promoteur) a été effectué sur les clones obtenus à la suite de ce troisième tour de shuffling. L'activité de ces clones a été mesurée à 30°C et à 50°C.

Dans ces conditions de criblage, le clone 17E5 (de séquence d'acides aminés SEQ ID N°1, codé par la séquence d'acides nucléiques SEQ ID N°2) a été sélectionné car il présente un ratio d'activité 30°C/50°C intéressant.

Le tableau 6 présente les activités relatives obtenues à 50°C et à 30°C pour le clone 17E5 et pour le clone 100B11 (clone référence issu du deuxième tour de shuffling).

**TABLEAU 6 : Activités relatives à 30°C**

| | **50°C** | **30°C** |
|---|---|---|
| **17E5** | 100% | 80% |
| **100B11** | 100% | 53% |

Les résultats montrent que le clone 17E5 conserve 80% d'activité à 30°C par rapport à son activité à 50°C contre 53% pour le clone 100B11.

De plus, son activité spécifique est supérieure d'un facteur 2 à celle de l'enzyme 100B11.

### 2-Détermination de l'activité beta-slucosidase.

Afin de déterminer le kcat relatif, l'activité du clone 17E5 a été mesurée à 30°C et à 50°C par le test d'activité tel que décrit précédemment.

Le tableau 7 présente la valeur du kcat ainsi que le facteur d'amélioration obtenus pour le clone 17E5 dans ces conditions expérimentales.

**TABLEAU 7 : Amélioration de l'activité beta-glucosidase à 30°C (résultats des cultures non induites)**

| | **kcat (min⁻¹)** | | **amélioration** | |
|---|---|---|---|---|
| | **30°C** | **50°C** | **30°C** | **50°C** |
| **17E5** | 4,2 | 10,94 | 2,32 | 2,17 |
| **100B11** | 1,81 | 5,03 | | |

Les résultats montrent une amélioration de l'activité enzymatique du clone 17E5 d'un facteur 2 par rapport au clone de référence et ce aux deux températures.

### EXEMPLE 4 : Expression des variants améliorés de beta-slucosîdases dans Trichoderma reesei

Le gène 17E5, a été cloné dans un vecteur permettant l'expression dans une souche *Trichoderma reesei* dérivée de la RUT C30 (ATCC 56765), CL847 (Durand et al, Enzyme Microb. Technol., 1988; 10:341-346) avec sélection par l'hygromycine (gène Hph de *Streptomyces hygroscopicus).* Le gène 17E5 a été placé sous contrôle d'un promoteur fort *cbhl,* inductible en même temps que les autres cellulases de *T. reesei.*

La transformation de *Trichoderma reesei* a été réalisée selon les méthodes classiques connues de l'homme de l'art (transformation de protoplastes par choc calcique et sélection à l'hygromycine 50µg/mL). Les transformants ont été purifiés par sporulation puis repiqués deux fois en milieu sélectif pour élimination des clones instables.

Trente clones ont ensuite été évalués en production de cellulases en plaques de 24 puits. Quelques spores de chaque clone ont été utilisés pour ensemencer 2 mL d'un milieu ayant la composition suivante : lactose 20g/L, cellulose Solka floc 20g/L, Peptone 5g/L, KH₂PO₄15g/L, (NH₄)₂SO₄ 5g/L, CaCl₂ 0,6g/L, MgSO₄ 0,6g, FeSO₄ 0,00 MnSO₄ 0,0014g/L, ZnSO₄ 0,0014g/L, CoCl₂ 0,0037 g/L, 11,6 g/L d'acide maléique, 12,1 g/L de Tris et 2,08 g/L de NaOH. Les fioles ont été mises à incuber à 30°C avec agitation à 150 rpm.

Au bout de 5 jours, les cultures ont été centrifugées et la concentration en protéines du surnageant mesurée par la méthode de Folin. L'activité beta-glucosidase des surnageants a été mesurée par hydrolyse du substrat chromophore para-nitrophényl beta-D-glucopyranoside (pNPG) dans les conditions suivantes:
- 50 mM de tampon citrate à pH 4,8
- 5 mM de pNPG
- 10 µL d'échantillon
- incubation à **30°C** pendant 30 min.

La réaction a été arrêtée par ajout de 100 µL de carbonate de sodium à 2%. La quantité de para-nitrophénol libéré par hydrolyse du pNPG a été mesurée par mesure de l'absorbance à 410 nm et comparée à une gamme de para-nitrophénol. La réaction était linéaire de 25 à 400 µM de para-nitrophénol. Les échantillons ont éventuellement été dilués pour que l'absorbance mesurée reste dans la linéarité de la gamme. L'activité beta-glucosidase a également été mesurée à 50°C, dans les mêmes conditions que ci-dessus, pour comparaison. Les clones présentant l'activité beta-glucosidase la plus importante (supérieure au moins d'un facteur 5 par rapport à la souche d'origine) ont été sélectionnés.

Le tableau 8 montre les activités beta-glucosidase pNPase 35°C/50°C mesurés en µmol/min/mg d'enzyme pour des surnageants issus respectivement d'une souche CL847 sauvage, d'une souche exprimant le variant 100B11 et d'un des clones exprimant le variant 17E5 obtenus selon la méthode décrite plus haut.

**Tableau 8: Activités beta-glucosidase de CL847 sauvage, du polypeptide 100B11 et du polypeptide 17E5**

| | Ratio d'activité 30°C/50°C | Activité spécifique à 30°C | Activité spécifique à 50°C |
|---|---|---|---|
| CL847 | 0,2 | 0,06 | 0,3 |
| 100B11 | 0,3 | 3,7 | 12,5 |
| 17E5 | 0,5 | 4,7 | 9,5 |

On constate une augmentation du ratio 30°C/50°C chez le clone 17E5, avec une activité spécifique supérieure à celle du variant 100B11 à la température de 30°C.

### EXEMPLE 5 : Expression recombinante de la beta-slucosidase sauvage (BGL1) et des variants améliorés 100B11 et 17E5 chez Saccharomyces cerevisiae

### 1- Production des protéines BGL1, 100B11 et 17E5 dans le cytoplasme des levures:

Le gène de la beta-glucosidase sauvage de *Trichoderma reesei* (BGL1) ainsi que ceux des variants 100B11 et 17E5 ont été clonés sans peptide signal dans le vecteur pESC-Leu (Agilent Technologies). Cette construction permet l'expression de la protéine dans le cytoplasme de la souche *Saccharomyces cerevisiae* EBY100, auxotrophe pour la leucine et le tryptophane (Boder ET et Wittrup KD, Biotechnol Prog, 1998, 14:55-62). Ce plasmide permet de placer l'expression des gènes sous le contrôle du promoteur GAL1 inductible au galactose, et possède le gène marqueur de sélection de l'auxotrophie (Leu2) qui permet la sélection des transformants. La protéine produite est également fusionnée au tag c-myc en N-terminal, permettant la détection et la purification de l'enzyme produite par chromatographie d'affinité.

La transformation de *S. cerevisiae* EBY100 a été réalisée selon les méthodes classiques connues de l'homme de l'art (transformation de levures par choc thermique et acétate de lithium). Les transformants ont été sélectionnés sur milieu YNB-Glc-Trp contenant 0,67% de Yeast Nitrogen Base (YNB), 2% de glucose et 0,01% de tryptophane.

Un transformant pour chaque gène (Sc-BGL1, Sc-100B11 et Sc-17E5) a été utilisé pour ensemencer 15 mL d'un milieu minimum YNB-Glc-CAA-Trp contenant 0,67% de YNB, 0,5% de casamino acid (CAA), 0,01% de tryptophane et 2% de glucose. Après 24h de préculture à 30°C avec agitation à 220 rpm, les 3 souches Sc-BGL1, Sc-100B11 et Sc-17E5 ont été utilisées pour ensemencer (à une DO₆₀₀ de 0,5) 150 mL de milieu YNB-Gal-CAA-Trp contenant 0,67% de YNB, 0,5% de CAA, 0,01% de tryptophane et 2% de galactose. Les cultures ont été incubées à 25°C sous agitation à 220 rpm.

Après 4 jours d'incubation, 20 mL de culture ont été centrifugés à 3 000 g, à 4°C pendant 5 min. Les culots de levures ont été repris dans 3 mL de tampon citrate 50 mM pH 5 et lysés mécaniquement avec une pression de 2,5 kbar. L'extrait cytoplasmique a été obtenu après centrifugation pendant 30 min à 50 000 g à 4°C.

### 2- Détermination de l'activité beta-slucosîdase

La concentration de protéines totales dans l'extrait cytoplasmique a été estimée en moyenne par dosage de Bradford (Bradford MM., Anal Biochem, 1976, 72:248-54) à 1,7 mg/mL.

L'activité beta-glucosidase des extraits cytoplasmiques a été mesurée par hydrolyse du substrat para-nitrophényl beta-D-glucopyranoside (pNPG) dans un volume de 600 µL dans les conditions suivantes:
- 50 mM de tampon citrate à pH 5
- 5 mM de pNPG
- 3,6 µL d'extrait cytoplasmique contenant 6,1 µg de protéines totales
- Incubation à 30°C ou 50°C pendant 30 min

La réaction a été arrêtée en ajoutant 100 µL de carbonate de sodium à 1M à 100 µL de réaction d'hydrolyse. La concentration en para-nitrophénol (pNP) libéré par hydrolyse du pNPG a été déterminée par mesure de l'absorbance à 415 nm et comparée à une gamme étalon du para-nitrophénol (linéaire de 0,36 µM à 360 µM). Les extraits cytoplasmiques ont éventuellement été dilués pour être en condition de vitesse initiale de réaction.

Le tableau 9 montre les ratios d'activité beta-glucosidase 30°C/50°C mesurés en µmol.min⁻¹.mg⁻¹ de protéines totales pour des extraits cytoplasmiques issus respectivement d'une souche exprimant l'enzyme sauvage (Sc-BGL1), d'une souche exprimant 100B11 (Sc-100B11) et d'une souche exprimant 17E5 (Sc-17E5).

**Tableau 9: Activités beta-glucosidase de Sc-BGL1, Sc-100B11 et Sc-17E5**

| | **Activité spécifique à 30°C** | **Activité spécifique à 50°C** | **Ratio d'activité 30°C/50°C** | **Amélioration de l'activité spécifique à 30°C par rapport à BGL1 sauvage** |
|---|---|---|---|---|
| **Sc-BGL1** | 0,15 | 0,41 | 0,4 | - |
| **Sc-100B11** | 0,18 | 0,64 | 0,3 | 1,2 |
| **Sc-17E5** | 0,46 | 1,12 | 0,4 | 3,1 |

Les résultats montrent que l'activité spécifique à 30°C de la souche Sc-17E5 est supérieure d'un facteur 3 par rapport à la souche Sc-BGL1 et d'un facteur 2,5 par rapport à Sc-100B11.

### EXEMPLE 6 : Purification et caractérisation de la beta-slucosidase sauvage (BGL1) et des variants améliorés 100B11 et 17E5 produits chez S. cerevisiae

### 1- Purification des beta-glucosidases:

Les extraits cytoplasmiques de Sc-BGL1 et des variants Sc-100B11 et Sc-17E5 de l'exemple 5 ont été utilisés pour purifier les enzymes correspondantes, BGL1, 100B11 et 17E5 selon le protocole suivant:
500 µL d'extrait cytoplasmique ont été incubés avec 20 µL de résine "Anti-c-Myc tag Gel" (MBL) pendant 1h à 4°C avec agitation axiale. Après 10 secondes de centrifugation à 13 000 rpm, la résine a été lavée 3 fois avec du tampon PBS 1X. Après incubation de la résine pendant 5 min à 4°C dans une solution d'élution composée du peptide c-myc (EQKLISEEDL) à 1 mg.mL⁻¹, l'élution de la protéine a été réalisée en centrifugeant 10 secondes à 13 000 rpm.

### 2- Détermination de l'activité beta-slucosidase

La concentration des enzymes purifiées est obtenue par mesure de l'absorbance à 280 nm au nanodrop, en utilisant un coefficient d'extinction molaire égale à 120 125 M⁻¹.cm⁻¹ pour BGL1 native et 120 250 M⁻¹.cm⁻¹ pour 100B11 et 17E5. Elle est en moyenne égale à 0,19 mg/mL.

La pureté de chaque enzyme a été vérifiée par électrophorèse sur gel de polyacrylamide à 10% en présence de SDS avec coloration des protéines au Bleu de Coomassie.

L'activité de BGL1 et des variants 100B11 et 17E5 purifiés a été mesurée à 30°C et à 50°C comme décrit précédemment.

Le tableau 10 montre les activités spécifiques de chaque enzyme (en µmol.min⁻¹.mg⁻¹ d'enzyme) déterminées lors de l'hydrolyse du pNPG à 30°C et 50°C.

**Tableau 10: Activités beta-glucosidase de BGL1, 100B11 et 17E5 purifiées**

| | **Activité spécifique à 30°C** | **Activité spécifique à 50°C** | **Ratio d'activité 30°C/50°C** | **Amélioration de l'activité spécifique à 30°C par rapport à BGL1 sauvage** |
|---|---|---|---|---|
| **BGL1** | 5,1 | 8,9 | 0,57 | - |
| **100B11** | 7,1 | 17,2 | 0,41 | 1,4 |
| **17E5** | 10,2 | 23,6 | 0,43 | 2,0 |

Les résultats montrent une amélioration à 30°C de l'activité spécifique du variant 17E5 d'un facteur 2 par rapport à BGL1 sauvage et de 1,4 par rapport au variant 100B11.

## Revendications

1. Polypeptide ayant une activité beta-glucosidase de séquence d'acides aminés SEQ ID N°1.

2. Acide nucléique purifié ou isolé, **caractérisé en ce qu'**il code le polypeptide selon la revendication **1 .**

3. Acide nucléique selon la revendication **2, caractérisé en ce qu'**il comprend la séquence d'acides nucléiques SEQ ID N°2.

4. Vecteur **caractérisé en ce qu'**il comprend un acide nucléique selon les revendications **2 ou 3.**

5. Cellule hôte isolée **caractérisée en ce qu'**elle comprend le polypeptide selon la revendication **1,** ou l'acide nucléique selon la revendication **2** ou le vecteur selon la revendication **4.**

6. Cellule hôte isolée selon la revendication **5, caractérisée en ce qu'**elle est choisie parmi *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* et *Saccharomyces.*

7. Cellule hôte isolée selon la revendication **5 ou 6, caractérisée en ce qu'**elle est choisie parmi *Trichoderma reesei, Trichoderma viridae, Trichoderma koningü, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckü, Clostridium butylicum, Pichia pastoris, Yarrowia lipolityca, Saccharomyces cerevisiae* et leurs mélanges.

8. Cellule hôte isolée selon la revendication **6, caractérisée en ce qu'**elle est de l'espèce *Trichoderma reesei.*

9. Cellule hôte isolée selon la revendication **6, caractérisée en ce qu'**elle est de l'espèce *Saccharomyces cerevisiae.*

10. Utilisation dudit polypeptide selon la revendication **1** ou d'une cellule selon l'une quelconque des revendications **5 à 9** pour l'hydrolyse des beta-oligosaccharides.

11. Utilisation dudit polypeptide selon la revendication **1** ou d'une cellule selon l'une quelconque des revendications **5 à 9** pour l'hydrolyse du cellobiose en glucose.

12. Utilisation dudit polypeptide selon la revendication **1** ou d'une cellule selon l'une quelconque des revendications **5 à 9** pour la production de biocarburant.

13. Composition enzymatique agissant sur la biomasse lignocellulosique, ladite composition enzymatique étant produite par des champignons filamenteux et comprenant au moins un polypeptide selon la revendication **1.**

14. Procédé de production de biocarburant à partir de la biomasse, comprenant les étapes suivantes :
- mise en suspension en phase aqueuse du matériau à hydrolyser ;
- hydrolyse en présence d'une composition enzymatique selon la revendication 13 ou d'une cellule selon l'une quelconque des revendications **5 à 9** de la biomasse lignocellulosique de manière à produire un hydrolysat contenant du glucose;
- fermentation du glucose de l'hydrolysat de manière à produire un moût de fermentation;
- séparation du biocarburant du moût de fermentation,
les étapes d'hydrolyse et de fermentation étant conduites de manière simultanée.

## Patentansprüche

1. Polypeptid mit einer Beta-Glucosidase Aktivität der Aminosäuresequenz SEQ ID NO 1.

2. Gereinigte oder isolierte Nucleinsäure, **dadurch gekennzeichnet, dass** sie das Polypeptid nach Anspruch **1** kodiert.

3. Nucleinsäure nach Anspruch **2, dadurch gekennzeichnet, dass** sie die Nucleinsäuresequenz SEQ ID NO 2 umfasst.

4. Vektor, **dadurch gekennzeichnet, dass** er eine Nucleinsäure nach Anspruch **2** oder **3** umfasst.

5. Isolierte Wirtszelle, **dadurch gekennzeichnet, dass** sie das Polypeptide nach Anspruch **1** umfasst, oder die Nucleinsäure nach Anspruch **2** oder den Vektor nach Anspruch **4.**

6. Isolierte Wirtszelle nach Anspruch **5, dadurch gekennzeichnet, dass** sie gewählt ist aus *Richoderma, Aspergillus, Eurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* und *Saccharomyces.*

7. Isolierte Wirtszelle nach Anspruch **5 oder 6, dadurch gekennzeichnet, dass** sie gewählt ist aus *Trichoderma reesei, Richoderma viridae, Richoderma koningü, Aspergillus niger, Aspergillus nidulans, Aspergillus wentii, Aspergillus oryzae, Aspergillus phoenicis, Eurospora crassa, Humicola grisae, Myceliophthora thermopile, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca, Saccharomyces cerevisiae* und ihren Mischungen.

8. Isolierte Wirtszelle nach Anspruch **6, dadurch gekennzeichnet, dass** sie von der Spezies *Trichoderma reesei* stammt.

9. Isolierte Wirtszelle nach Anspruch **6, dadurch gekennzeichnet, dass** sie von der Spezies *Saccharomyces cerevisiae* stammt.

10. Verwendung des Polypeptids nach Anspruch **1** oder einer Zelle nach einem der Ansprüche **5 bis 9** für die Hydrolyse der Beta-Oligosaccharide.

11. Verwendung des Polypeptids nach Anspruch **1** oder einer Zelle nach einem der Ansprüche **5 bis 9** für die Hydrolyse der Glucose-Cellobiose.

12. Verwendung des Polypeptids nach Anspruch **1** oder einer Zelle nach einem der Ansprüche **5 bis 9** für die Produktion von Biokraftstoff.

13. Enzymatische Zusammensetzung, die auf Lignocellulose-Biomasse wirkt, wobei die enzymatische Zusammensetzung durch filamentöse Pilze hergestellt wird und wenigstens ein Polypeptid nach Anspruch **1** umfasst.

14. Verfahren zur Herstellung von Biokraftstoff aus Biomasse, welches die folgenden Schritte umfasst:
- Suspendieren des zu hydrolysierenden Materials in wässriger Phase;
- Hydrolyse in Anwesenheit einer enzymatischen Zusammensetzung nach Anspruch 13 oder einer Zelle nach einem der Ansprüche **5 bis 9** der Lignocellulose-Biomasse zum Herstellen eines Glucose-haltigen Hydrolysats;
- Fermentierung der Glucose des Hydrolysats zum Herstellen eines Fermentierungs-Mosts;
- Trennen des Biokraftstoffes von dem Fermentierungs-Most,
wobei die Schritte der Hydrolyse und der Fermentierung gleichzeitig durchgeführt werden.

## Claims

1. Polypeptide which has beta-glucosidase activity, of amino acid sequence SEQ ID No. 1.

2. Purified or isolated nucleic acid, **characterized in that** it encodes the polypeptide according to Claim **1.**

3. Nucleic acid according to Claim **2, characterized in that** it comprises the nucleic acid sequence SEQ ID No. 2.

4. Vector **characterized in that** it comprises a nucleic acid according to Claim **2 or 3.**

5. Isolated host cell **characterized in that** it comprises the polypeptide according to Claim **1,** or the nucleic acid according to Claim 2 or the vector according to Claim 4.

6. Isolated host cell according to Claim **5, characterized in that** it is chosen from *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* and *Saccharomyces.*

7. Isolated host cell according to Claim **5 or 6, characterized in that** it is chosen from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca, Saccharomyces cerevisiae,* and mixtures thereof.

8. Isolated host cell according to Claim **6, characterized in that** it is the species *Trichoderma reesei.*

9. Isolated host cell according to Claim **6, characterized in that** it is the species *Saccharomyces cerevisiae.*

10. Use of said polypeptide according to Claim **1** or of a cell according to any one of Claims **5 to 9,** for the hydrolysis of beta-oligosaccharides.

11. Use of said polypeptide according to Claim **1** or of a cell according to any one of Claims **5 to 9,** for the hydrolysis of cellobiose to glucose.

12. Use of said polypeptide according to Claim **1** or of a cell according to any one of Claims **5 to 9,** for the production of biofuel.

13. Enzymatic composition which acts on lignocellulosic biomass, said enzymatic composition being produced by filamentous fungi and comprising at least one polypeptide according to Claim **1.**

14. Process for producing biofuel from biomass, comprising the following steps:
- suspension, in an aqueous phase, of the material to be hydrolyzed;
- hydrolysis, in the presence of an enzymatic composition according to Claim 13 or of a cell according to any one of Claims **5 to 9,** of the lignocellulosic biomass so as to produce a hydrolysate containing glucose;
- fermentation of the glucose of the hydrolysate so as to produce a fermentation must;
- separation of the biofuel from the fermentation must,
the hydrolysis and fermentation steps being carried out simultaneously.


### SEQUENCE LISTING

<110> IFP Energies nouvelles
<120> Polypeptide à activité beta-glucosidase renforcée à basse température
<130> BFF120334
<160> 12
<170> BiSSAP 1.0
<210> 1
   <211> 744
   <212> PRT
   <213> Trichoderma reesei
<400> 1
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Gly Tyr Pro Gln Leu Cys Leu Gln Asp Gly Pro Leu Gly
                   85                  90                  95
   Ile Arg Phe Gly Gly Ser Val Thr Ala Phe Thr Pro Gly Ile Gln Ala
               100                 105                 110
   Ala Ser Thr Trp Asp Thr Glu Leu Met Arg Gln Arg Gly Glu Tyr Leu
           115                 120                 125
   Gly Ala Glu Ala Lys Gly Cys Gly Ile His Val Leu Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Ala Glu Thr Ile
                   165                 170                 175
   Glu Gly Leu Gln Ser Ala Gly Val Gln Ala Cys Ala Lys His Tyr Ile
               180                 185                 190
   Val Asn Glu Gln Glu Leu Asn Arg Glu Thr Ile Ser Ser Asn Pro Asp
           195                 200                 205
   Asp Arg Thr Leu His Glu Leu Tyr Leu Trp Pro Phe Ala Asp Ala Val
       210                 215                 220
   His Ala Asn Val Ala Ser Val Met Cys Ser Tyr Asn Lys Ile Asn Gly
   225                 230                 235                 240
   Ser Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 2
   <211> 2235
   <212> DNA
   <213> Trichoderma reesei
<400> 2
   atgcgttacc gaacagcagc tgcgctggca cttgccactg ggccctttgc tagggcagac      60
   agtcactcaa catcgggggc ctcggctgag gcagttgtac ctcctgcagg gactccatgg     120
   ggaaccgcgt acgacaaggc gaaggccgca ttggcaaagc tcaatctcca agataaggtc     180
   ggcatcgtga gcggtgtcgg ctggaacggc ggtccttgcg ttggaaacac atctccggcc     240
   tccaagatcg gctatccaca gctatgcctt caagacggac ccctcggtat ccgattcggc     300
   ggcagcgtca cagcctttac gccgggcatc caagcggcct cgacgtggga taccgagttg     360
   atgcgccagc gtggagagta cctgggtgcc gaggccaagg gctgcgggat tcatgtcctg     420
   cttggtcctg tggctgggcc gctgggaaag actccgcagg gcggtcgcaa ctgggagggc     480
   ttcggtgtcg atccatatct cacgggcatt gccatggccg agacaatcga gggcctgcag 540
   tcggccggcg tgcaggcgtg cgcgaagcac tatatcgtca acgagcagga gctcaatcga 600
   gaaaccattt cgagcaaccc agatgaccga actctccatg agctgtatct gtggccattt 660
   gccgacgcgg ttcacgccaa tgtcgcttct gtcatgtgct cgtacaacaa gatcaatggc 720
   agctgggcct gcgaggatca gtacacgctg cagactgtgc tgaaagacca gctggggttc 780
   ccaggctatg tcatgacgga ctggaacgca cagcacacga ctgtccaaag cgcgaattct 840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 3
   <211> 744
   <212> PRT
   <213> Trichoderma reesei
<400> 3
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Val Arg Tyr Ser Thr Gly Ser Thr Ala Phe Thr Pro Gly Val Gln Ala
               100                 105                 110
   Ala Ser Thr Trp Asp Val Asn Leu Ile Arg Glu Arg Gly Gln Phe Ile
           115                 120                 125
   Gly Glu Glu Val Lys Ala Ser Gly Ile His Val Ile Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Gly Gln Thr Ile
                   165                 170                 175
   Asn Gly Ile Gln Ser Val Gly Val Gln Ala Thr Ala Lys His Tyr Ile
               180                 185                 190
   Leu Asn Glu Gln Glu Leu Asn Arg Glu Thr Ile Ser Ser Asn Pro Asp
           195                 200                 205
   Asp Arg Thr Leu His Glu Leu Tyr Thr Trp Pro Phe Ala Asp Ala Val
       210                 215                 220
   Gln Ala Asn Val Ala Ser Val Met Cys Ser Tyr Asn Lys Val Asn Thr
   225                 230                 235                 240
   Thr Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                     535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 4
   <211> 2235
   <212> DNA
   <213> Trichoderma reesei
<400> 4
   atgcgttacc gaacagcagc tgcgctggca cttgccactg ggccctttgc tagggcagac     60
   agtcactcaa catcgggggc ctcggctgag gcagttgtac ctcctgcagg gactccatgg    120
   ggaaccgcgt acgacaaggc gaaggccgca ttggcaaagc tcaatctcca agataaggtc    180
   ggcatcgtga gcggtgtcgg ctggaacggc ggtccttgcg ttggaaacac atctccggcc    240
   tccaagatca gctatccatc gctatgcctt caagacggac ccctcggtgt tcgatactcg    300
   acaggcagca cagcctttac gccgggcgtt caagcggcct cgacgtggga tgtcaatttg    360
   atccgcgaac gtggacagtt catcggtgag gaggtgaagg cctcggggat tcatgtcata    420
   cttggtcctg tggctgggcc gctgggaaag actccgcagg gcggtcgcaa ctgggagggc    480
   ttcggtgtcg atccatatct cacgggcatt gccatgggtc aaaccatcaa cggcatccag    540
   tcggtaggcg tgcaggcgac agcgaagcac tatatcctca acgagcagga gctcaatcga    600
   gaaaccattt cgagcaaccc agatgaccga actctccatg agctgtatac ttggccattt    660
   gccgacgcgg ttcaggccaa tgtcgcttct gtcatgtgct cgtacaacaa ggtcaatacc    720
   acctgggcct gcgaggatca gtacacgctg cagactgtgc tgaaagacca gctggggttc    780
   ccaggctatg tcatgacgga ctggaacgca cagcacacga ctgtccaaag cgcgaattct    840
   gggcttgaca tgtcaatgcc tggcacagac ttcaacggta acaatcggct ctggggtcca    900
   gctctcacca atgcggtaaa tagcaatcag gtccccacga gcagagtcga cgatatggtg    960
   actcgtatcc tcgccgcatg gtacttgaca ggccaggacc aggcaggcta tccgtcgttc   1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 5
   <211> 744
   <212> PRT
   <213> Trichoderma reesei
<400> 5
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Ile Arg Phe Gly Thr Gly Ser Thr Ala Phe Thr Pro Gly Val Gln Ala
               100                 105                 110
   Ala Ser Thr Trp Asp Thr Glu Leu Met Arg Gln Arg Gly Glu Tyr Leu
           115                 120                 125
   Gly Ala Glu Ala Lys Gly Cys Gly Ile His Val Leu Leu Gly Pro Val
           130                     135                     140
   Ala Gly Pro Leu Gly Lys Thr Pro Gln Gly Gly Arg Asn Trp Glu Gly
   145                 150                     155                 160
   Phe Gly Val Asp Pro Tyr Leu Thr Gly Ile Ala Met Ala Glu Thr Ile
                   165                     170                 175
   Glu Gly Leu Gln Ser Ala Gly Val Gln Ala Cys Ala Lys His Tyr Ile
               180                     185                 190
   Val Asn Glu Gln Glu Leu Asn Arg Glu Thr Ile Ser Ser Asp Val Asp
           195                     200                 205
   Asp Arg Thr Met His Glu Leu Tyr Leu Trp Pro Phe Ala Asp Ala Val
       210                     215                 220
   Gln Ala Asn Val Ala Ser Val Met Cys Ser Tyr Asn Lys Ile Asn Gly
   225                 230                     235                 240
   Ser Trp Ala Cys Glu Asp Gln Tyr Thr Leu Gln Thr Val Leu Lys Asp
                   245                     250                 255
   Gln Leu Gly Phe Pro Gly Tyr Val Met Thr Asp Trp Asn Ala Gln His
               260                     265                 270
   Thr Thr Val Gln Ser Ala Asn Ser Gly Leu Asp Met Ser Met Pro Gly
           275                     280                 285
   Thr Asp Phe Asn Gly Asn Asn Arg Leu Trp Gly Pro Ala Leu Thr Asn
       290                     295                 300
   Ala Val Asn Ser Asn Gln Val Pro Thr Ser Arg Val Asp Asp Met Val
   305                 310                     315                 320
   Thr Arg Ile Leu Ala Ala Trp Tyr Leu Thr Gly Gln Asp Gln Ala Gly
                   325                     330                 335
   Tyr Pro Ser Phe Asn Ile Ser Arg Asn Val Gln Gly Asn His Lys Thr
               340                     345                 350
   Asn Val Arg Ala Ile Ala Arg Asp Gly Ile Val Leu Leu Lys Asn Asp
           355                     360                 365
   Ala Asn Ile Leu Pro Leu Lys Lys Pro Ala Ser Ile Ala Val Val Gly
       370                     375                 380
   Ser Ala Ala Ile Ile Gly Asn His Ala Arg Asn Ser Pro Ser Cys Asn
   385                 390                     395                 400
   Asp Lys Gly Cys Asp Asp Gly Ala Leu Gly Met Gly Trp Gly Ser Gly
                   405                     410                 415
   Ala Val Asn Tyr Pro Tyr Phe Val Ala Pro Tyr Asp Ala Ile Asn Thr
               420                     425                 430
   Arg Ala Ser Ser Gln Gly Thr Gln Val Thr Leu Ser Asn Thr Asp Asn
           435                     440                 445
   Thr Ser Ser Gly Ala Ser Ala Ala Arg Gly Lys Asp Val Ala Ile Val
       450                     455                 460
   Phe Ile Thr Ala Asp Ser Gly Glu Gly Tyr Ile Thr Val Glu Gly Asn
   465                 470                     475                 480
   Ala Gly Asp Arg Asn Asn Leu Asp Pro Trp His Asn Gly Asn Ala Leu
                   485                     490                 495
   Val Gln Ala Val Ala Gly Ala Asn Ser Asn Val Ile Val Val Val His
               500                     505                 510
   Ser Val Gly Ala Ile Ile Leu Glu Gln Ile Leu Ala Leu Pro Gln Val
           515                     520                 525
   Lys Ala Val Val Trp Ala Gly Leu Pro Ser Gln Glu Ser Gly Asn Ala
       530                     535                 540
   Leu Val Asp Val Leu Trp Gly Asp Val Ser Pro Ser Gly Lys Leu Val
   545                 550                     555                 560
   Tyr Thr Ile Ala Lys Ser Pro Asn Asp Tyr Asn Thr Arg Ile Val Ser
                   565                     570                 575
   Gly Gly Ser Asp Ser Phe Ser Glu Gly Leu Phe Ile Asp Tyr Lys His
               580                     585                 590
   Phe Asp Asp Ala Asn Ile Thr Pro Arg Tyr Glu Phe Gly Tyr Gly Leu
           595                     600                 605
   Ser Tyr Thr Lys Phe Asn Tyr Ser Arg Leu Ser Val Phe Ser Thr Ala
       610                     615                 620
   Lys Ser Gly Pro Ala Thr Gly Ala Val Val Pro Gly Gly Pro Ser Asp
   625                 630                     635                 640
   Leu Phe Gln Asn Val Ala Thr Val Thr Val Asp Ile Ala Asn Ser Gly
                   645                     650                 655
   Gln Val Thr Gly Ala Glu Val Ala Gln Leu Tyr Ile Thr Tyr Pro Ser
               660                     665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 6
   <211> 2235
   <212> DNA
   <213> Trichoderma reesei
<400> 6
   atgcgttacc gaacagcagc tgcgctggca cttgccactg ggccctttgc tagggcagac      60
   agtcactcaa catcgggggc ctcggctgag gcagttgtac ctcctgcagg gactccatgg     120
   ggaaccgcgt acgacaaggc gaaggccgca ttggcaaagc tcaatctcca agataaggtc     180
   ggcatcgtga gcggtgtcgg ctggaacggc ggtccttgcg ttggaaacac atctccggcc     240
   tccaagatca gctatccatc gctatgcctt caagacggac ccctcggtat ccgattcggc     300
   acaggcagca cagcctttac gccgggcgtt caagcggcct cgacgtggga taccgagttg     360
   atgcgccagc gtggagagta cctgggtgcc gaggccaagg gctgcgggat tcatgtcctg     420
   cttggtcctg tggctgggcc gctgggaaag actccgcagg gcggtcgcaa ctgggagggc     480
   ttcggtgtcg atccatatct cacgggcatt gccatggccg agacaatcga gggcctgcag     540
   tcggccggcg tgcaggcgtg cgcgaagcac tatatcgtca acgagcagga gctcaatcga     600
   gaaaccattt cgagcgacgt cgatgaccga actatgcatg agctgtatct gtggccattt     660
   gccgacgcgg ttcaggccaa tgtcgcttct gtcatgtgct cgtacaacaa gatcaatggc     720
   agctgggcct gcgaggatca gtacacgctg cagactgtgc tgaaagacca gctggggttc     780
   ccaggctatg tcatgacgga ctggaacgca cagcacacga ctgtccaaag cgcgaattct     840
   gggcttgaca tgtcaatgcc tggcacagac ttcaacggta acaatcggct ctggggtcca     900
   gctctcacca atgcggtaaa tagcaatcag gtccccacga gcagagtcga cgatatggtg     960
   actcgtatcc tcgccgcatg gtacttgaca ggccaggacc aggcaggcta tccgtcgttc    1020
   aacatcagca gaaatgttca aggaaaccac aagaccaatg tcagggcaat tgccagggac    1080
   ggcatcgttc tgctcaagaa tgacgccaac atcctgccgc tcaagaagcc cgctagcatt    1140
   gccgtcgttg gatctgccgc aatcattggt aaccacgcca gaaactcgcc ctcgtgcaac    1200
   gacaaaggct gcgacgacgg ggccttgggc atgggttggg gttccggcgc cgtcaactat    1260
   ccgtacttcg tcgcgcccta cgatgccatc aataccagag cgtcttcgca gggcacccag    1320
   gttaccttga gcaacaccga caacacgtcc tcaggcgcat ctgcagcaag aggaaaggac    1380
   gtcgccatcg tcttcatcac cgccgactcg ggtgaaggct acatcaccgt ggagggcaac    1440
   gcgggcgatc gcaacaacct ggatccgtgg cacaacggca atgccctggt ccaggcggtg    1500
   gccggtgcca acagcaacgt cattgttgtt gtccactccg ttggcgccat cattctggag    1560
   cagattcttg ctcttccgca ggtcaaggcc gttgtctggg cgggtcttcc ttctcaggag     1620
   agcggcaatg cgctcgtcga cgtgctgtgg ggagatgtca gcccttctgg caagctggtg     1680
   tacaccattg cgaagagccc caatgactat aacactcgca tcgtttccgg cggcagtgac     1740
   agcttcagcg agggactgtt catcgactat aagcacttcg acgacgccaa tatcacgccg     1800
   cggtacgagt tcggctatgg actgtcttac accaagttca actactcacg cctctccgtc     1860
   ttttcgaccg ccaagtctgg tcctgcgact ggggccgttg tgccgggagg cccgagtgat     1920
   ctgttccaga atgtcgcgac agtcaccgtt gacatcgcaa actctggcca agtgactggt     1980
   gccgaggtag cccagctgta catcacctac ccatcttcag cacccaggac ccctccgaag     2040
   cagctgcgag gctttgccaa gctgaacctc acgcctggtc agagcggaac agcaacgttc     2100
   aacatccgac gacgagatct cagctactgg gacacggctt cgcagaaatg ggtggtgccg     2160
   tcggggtcgt ttggcatcag cgtgggagcg agcagccggg atatcaggct gacgagcact     2220
   ctgtcggtag cgtag                                                      2235
<210> 7
   <211> 726
   <212> PRT
   <213> Chaetomium globosum
<400> 7
   Met Thr Thr Leu Arg Asn Phe Ala Leu Leu Ala Ala Ala Val Leu Ala
   1               5                   10                  15
   Arg Val Glu Ala Leu Glu Ala Ala Asp Trp Ala Ala Ala Glu Ala Ser
               20                  25                  30
   Ala Lys Thr Ala Leu Ala Lys Met Ser Gln Gln Asp Lys Ile Ser Ile
           35                  40                  45
   Val Thr Gly Ile Gly Trp Asp Lys Gly Pro Cys Val Gly Asn Thr Ala
       50                  55                  60
   Ala Ile Asn Ser Ile Asn Tyr Pro Gln Leu Cys Leu Gln Asp Gly Pro
   65                  70                  75                  80
   Leu Gly Ile Arg Phe Gly Thr Gly Ser Thr Ala Phe Thr Pro Gly Val
                   85                  90                  95
   Gln Ala Ala Ser Thr Trp Asp Thr Glu Leu Met Arg Gln Arg Gly Glu
               100                 105                 110
   Tyr Leu Gly Ala Glu Ala Lys Gly Cys Gly Ile His Val Leu Leu Gly
           115                 120                 125
   Pro Val Ala Gly Ala Leu Gly Lys Ile Pro His Gly Gly Arg Asn Trp
       130                 135                 140
   Glu Gly Phe Gly Thr Asp Pro Tyr Leu Ala Gly Ile Ala Met Ala Glu
   145                 150                 155                 160
   Thr Ile Glu Gly Leu Gln Ser Ala Gly Val Gln Ala Cys Ala Lys His
                   165                 170                 175
   Tyr Ile Val Asn Glu Gln Glu Leu Asn Arg Glu Thr Ile Ser Ser Asp
               180                 185                 190
   Val Asp Asp Arg Thr Met His Glu Leu Tyr Leu Trp Pro Phe Ala Asp
           195                 200                 205
   Ala Val His Ala Asn Val Ala Ser Val Met Cys Ser Tyr Asn Lys Ile
       210                 215                 220
   Asn Gly Ser Trp Gly Cys Glu Asn Asp His Ala Gln Asn Gly Leu Leu
   225                 230                 235                 240
   Lys Lys Glu Leu Gly Phe Lys Gly Tyr Val Val Ser Asp Trp Asn Ala
                   245                 250                 255
   Gln His Thr Thr Asp Gly Ala Ala Asn Asn Gly Met Asp Met Thr Met
               260                 265                 270
   Pro Gly Ser Asp Tyr Asn Gly Asn Asn Val Leu Trp Gly Pro Gln Leu
                   275                     280                     285
   Ser Asn Ala Val Asn Ser Asn Arg Val Ser Arg Asp Arg Leu Asp Asp
       290                     295                     300
   Met Ala Lys Arg Ile Leu Thr Ser Trp Tyr Leu Leu Gly Gln Asn Ser
   305                     310                     315                     320
   Gly Tyr Pro Asn Ile Asn Ile Asn Ala Asn Val Gln Gly Asn His Lys
                       325                     330                     335
   Glu Asn Val Arg Ala Val Ala Arg Asp Gly Ile Val Leu Leu Lys Asn
               340                     345                     350
   Asp Glu Gly Val Leu Pro Leu Lys Lys Pro Gly Lys Val Ala Leu Val
           355                     360                     365
   Gly Ser Ala Ala Ser Val Asn Ser Ala Gly Pro Asn Ala Cys Val Asp
       370                     375                     380
   Lys Gly Cys Asn Thr Gly Ala Leu Gly Met Gly Trp Gly Ser Gly Ser
   385                     390                     395                     400
   Val Asn Tyr Pro Tyr Phe Val Ala Pro Tyr Asp Ala Leu Lys Thr Arg
                       405                     410                     415
   Ala Gln Ala Asp Gly Thr Thr Leu Ser Leu His Asn Ser Asp Ser Thr
               420                     425                     430
   Asn Gly Val Ser Gly Val Val Ser Gly Ala Asp Val Ala Ile Val Val
           435                     440                     445
   Ile Thr Ala Asp Ser Gly Glu Gly Tyr Ile Thr Val Glu Gly His Ala
       450                     455                     460
   Gly Asp Arg Asn His Leu Asp Pro Trp His Asp Gly Asn Ala Leu Val
   465                     470                     475                     480
   Lys Ala Val Ala Ala Ala Asn Lys Asn Thr Ile Val Val Val His Ser
                       485                     490                     495
   Thr Gly Pro Ile Ile Leu Glu Thr Ile Leu Ala Thr Glu Gly Val Lys
               500                     505                     510
   Ala Val Val Trp Ala Gly Leu Pro Ser Gln Glu Asn Gly Asn Ala Leu
           515                     520                     525
   Val Asp Val Leu Tyr Gly Leu Thr Ser Pro Ser Gly Lys Leu Val Tyr
       530                     535                     540
   Ser Ile Ala Lys Arg Pro Glu Asp Tyr Gly Thr Ala Pro Ser Lys Gly
   545                     550                     555                     560
   Ser Asn Asp Lys Phe Thr Glu Gly Leu Phe Val Asp Tyr Arg His Phe
                       565                     570                     575
   Asp Asn Ala Lys Ile Glu Pro Arg Tyr Glu Phe Gly Phe Gly Leu Ser
               580                     585                     590
   Tyr Thr Glu Phe Thr Tyr Ala Asp Leu Ser Val Thr Ser Thr Val Thr
           595                     600                     605
   Ala Gly Pro Ala Ser Gly Glu Thr Ile Pro Gly Gly Ala Ala Asp Leu
       610                     615                     620
   Trp Glu Thr Val Ala Thr Val Thr Ala Ser Ile Thr Asn Ser Gly Glu
   625                     630                     635                     640
   Val Glu Gly Ala Glu Val Ala Gln Leu Tyr Ile Thr Leu Pro Ser Ala
                       645                     650                     655
   Ala Pro Ser Thr Pro Pro Lys Gln Leu Arg Gly Phe Ala Lys Leu Lys
               660                     665                     670
   Leu Glu Pro Gly Ala Ser Gly Val Ala Thr Phe Asn Leu Arg Arg Arg
           675                     680                     685
   Asp Leu Ser Tyr Trp Asp Ala Gly Arg Gly Gln Trp Val Val Pro Ala
       690                     695                     700
   Gly Glu Phe Thr Val Ser Val Gly Ala Ser Ser Arg Asp Val Arg Leu
   705                     710                     715                     720
   Thr Gly Ser Leu Thr Ala
                       725
<210> 8
   <211> 2181
   <212> DNA
   <213> Chaetomium globosum
<400> 8
   atgacgacgc tccgcaactt tgcgctgctc gcagcggcgg tgcttgcgcg ggtcgaggcc 60
   ctcgaggccg ccgactgggc tgcggctgag gcctcagcca aaaccgcact ggcaaagatg 120
   tcacaacaag acaaaatcag cattgtgacg ggcatcggct gggacaaggg tccctgtgtc 180
   ggcaacacgg ccgccatcaa ctcgatcaac tacccgcagc tctgcctaca ggacggcccg 240
   ctcgggatcc gcttcggcac cggctcgacg gccttcaccc cgggcgtcca agccgcctcg 300
   acatgggata ccgagctgat gcgccagcgc ggcgagtacc tcggggccga ggccaagggg 360
   tgcggcatcc acgtgttgct gggccccgtg gccggggcac tgggcaagat cccgcacggc 420
   gggcgcaact gggaaggatt cgggacggac ccgtacctgg cgggcatcgc catggccgag 480
   acgatcgagg ggctgcagtc ggcgggggtg caggcgtgcg ccaagcacta catcgtcaac 540
   gagcaggagc tcaaccgcga gaccatcagc agcgacgtcg acgaccgcac catgcacgag 600
   ctgtacctgt ggcccttcgc cgacgccgtg cacgccaacg tggccagcgt catgtgcagc 660
   tacaacaaga tcaacggctc gtggggctgc gagaacgacc acgcccaaaa cggcctgctc 720
   aagaaggagc tcggcttcaa gggttacgtc gtcagcgact ggaacgcgca gcacacgacc 780
   gacggcgccg ccaacaacgg catggacatg accatgccgg gcagcgacta caacggcaac 840
   aacgtgctct ggggcccgca gctcagcaac gccgtcaaca gcaaccgggt ctcgcgcgac 900
   cggctcgacg acatggccaa acgcatcctc acctcatggt acctcctggg ccagaactcg 960
   ggctacccca acatcaacat caacgccaac gtgcagggca accacaagga gaacgtgcgg 1020
   gcggtggcgc gcgacggcat cgtgctgctc aagaacgacg agggcgtgct cccgctgaag 1080
   aagccaggca aggtggctct cgtcggatcg gcggcctcgg tcaacagcgc gggccccaac 1140
   gcgtgcgtcg acaagggctg caacacgggc gcgctcggca tgggctgggg gtccgggtcc 1200
   gtcaactacc cctactttgt ggcgccctac gacgcgctca agacgcgcgc ccaggccgac 1260
   ggcaccacgc tcagcctgca caactcggac tcgaccaacg gcgtatcggg cgtggtgtcg 1320
   ggcgccgacg tggccatcgt ggtgatcacg gcggactcgg gcgagggcta catcacggtc 1380
   gagggccacg ccggcgaccg caaccacctg gacccgtggc acgacggcaa cgcgctggtt 1440
   aaggcggtgg ccgcggccaa caagaacacc atcgtggtag tgcacagcac agggcccatc 1500
   atcctcgaga ccatcctggc gacggagggt gtcaaggcgg ttgtgtgggc cggcctgccg 1560
   agtcaggaga acggcaacgc gctagttgac gttttgtacg gcctgacttc gccctcaggc 1620
   aaactggtct actccatcgc caagcgcccc gaggactatg gcacggcccc ctccaagggc 1680
   agtaacgaca agttcaccga aggcctgttt gtcgactacc ggcactttga caacgccaag 1740
   attgagccgc ggtacgagtt tggctttggt ttgtcctaca ccgaattcac ctacgccgac 1800
   ctctccgtca cttccaccgt aacggccggc cccgcctcag gcgagaccat acccggcggc 1860
   gcggccgacc tctgggagac tgtcgcaacg gtcacggcgt ccatcacgaa cagcggcgag 1920
   gtggagggcg ccgaggtggc gcagctgtac atcacgctgc cgtcggcggc cccctcgacg 1980
   ccgcccaagc agctgcgcgg gttcgccaag ctcaagctcg agccgggggc gtcgggcgtc 2040
   gcgaccttca acctgcgccg tcgcgatctg agttattggg atgccgggcg cggccagtgg 2100
   gtggtgccgg cgggcgagtt tacggtttcg gttggtgcga gttcgaggga tgtgcgcttg 2160
   acggggagct tgactgctta g 2181
<210> 9
   <211> 735
   <212> PRT
   <213> Neurospora crassa
<400> 9
   Met His Leu Arg Ile Phe Ala Val Leu Ala Ala Thr Ser Leu Ala Trp
   1               5                   10                  15
   Ala Glu Thr Ser Glu Lys Gln Ala Arg Gln Ala Gly Ser Gly Phe Ala
               20                  25                  30
   Ala Trp Asp Ala Ala Tyr Ser Gln Ala Ser Thr Ala Leu Ser Lys Leu
           35                  40                  45
   Ser Gln Gln Asp Lys Val Asn Ile Val Thr Gly Val Gly Trp Asn Lys
       50                  55                  60
   Gly Pro Cys Val Gly Asn Thr Pro Ala Ile Ala Ser Ile Gly Tyr Pro
   65                  70                  75                  80
   Gln Leu Cys Leu Gln Asp Gly Pro Leu Gly Ile Arg Phe Gly Gly Ser
                   85                  90                  95
   Val Thr Ala Phe Thr Pro Gly Ile Gln Ala Ala Ser Thr Trp Asp Val
               100                 105                 110
   Glu Leu Ile Arg Gln Arg Gly Val Tyr Leu Gly Ala Glu Ala Arg Gly
           115                 120                 125
   Val Gly Val His Val Leu Leu Gly Pro Val Ala Gly Ala Leu Gly Lys
       130                 135                 140
   Ile Pro Asn Gly Gly Arg Asn Trp Glu Gly Phe Gly Pro Asp Pro Tyr
   145                 150                 155                 160
   Leu Thr Gly Ile Ala Met Ser Glu Thr Ile Glu Gly Ile Gln Ser Asn
                   165                 170                 175
   Gly Val Gln Ala Cys Ala Lys His Phe Ile Leu Asn Glu Gln Glu Thr
               180                 185                 190
   Asn Arg Asp Thr Ile Ser Ser Val Val Asp Asp Arg Thr Met His Glu
           195                 200                 205
   Leu Tyr Leu Phe Pro Phe Ala Asp Ala Val His Ser Asn Val Ala Ser
       210                 215                 220
   Val Met Cys Ser Tyr Asn Lys Val Asn Gly Thr Trp Ala Cys Glu Asn
   225                 230                 235                 240
   Asp Lys Ile Gln Asn Gly Leu Leu Lys Lys Glu Leu Gly Phe Lys Gly
                   245                 250                 255
   Tyr Val Met Ser Asp Trp Asn Ala Gln His Thr Thr Asn Gly Ala Ala
               260                 265                 270
   Asn Ser Gly Met Asp Met Thr Met Pro Gly Ser Asp Phe Asn Gly Lys
           275                 280                 285
   Thr Ile Leu Trp Gly Pro Gln Leu Asn Thr Ala Val Asn Asn Gly Gln
       290                 295                 300
   Val Ser Lys Ala Arg Leu Asp Asp Met Ala Lys Arg Ile Leu Ala Ser
   305                 310                 315                 320
   Trp Tyr Leu Leu Glu Gln Asn Ser Gly Tyr Pro Ala Thr Asn Leu Lys
                   325                 330                 335
   Ala Asn Val Gln Gly Asn His Lys Glu Asn Val Arg Ala Val Ala Arg
               340                 345                 350
   Asp Gly Ile Val Leu Leu Lys Asn Asp Asp Asn Ile Leu Pro Leu Lys
           355                 360                 365
   Lys Pro Ser Lys Leu Ala Ile Ile Gly Ser Ser Ser Val Val Asn Pro
       370                 375                 380
   Ala Gly Arg Asn Ala Cys Thr Asp Arg Gly Cys Asn Thr Gly Ala Leu
   385                 390                 395                 400
   Gly Met Gly Trp Gly Ser Gly Thr Ala Asp Tyr Pro Tyr Phe Val Ala
                   405                 410                 415
   Pro Tyr Asp Ala Leu Lys Thr Arg Ala Gln Ser Asp Gly Thr Thr Val
               420                 425                 430
   Asn Leu Leu Ser Ser Asp Ser Thr Ser Gly Val Ala Asn Ala Ala Ser
           435                 440                 445
   Gly Ala Asp Ala Ala Leu Val Phe Ile Thr Ala Asp Ser Gly Glu Gly
           450                     455                 460
   Tyr Ile Thr Val Glu Gly Val Thr Gly Asp Arg Pro Asn Leu Asp Pro
   465                 470                 475                 480
   Trp His Asn Gly Asn Gln Leu Val Gln Ala Val Ala Gln Ala Asn Lys
                   485                 490                 495
   Asn Thr Ile Val Val Val His Ser Thr Gly Pro Ile Ile Leu Glu Thr
               500                 505                 510
   Ile Leu Ala Gln Pro Gly Val Lys Ala Val Val Trp Ala Gly Leu Pro
           515                 520                 525
   Ser Gln Glu Asn Gly Asn Ala Leu Val Asp Val Leu Tyr Gly Leu Val
       530                 535                 540
   Ser Pro Ser Gly Lys Leu Pro Tyr Thr Ile Ala Lys Ser Glu Ser Asp
   545                 550                 555                 560
   Tyr Gly Thr Ala Val Gln Arg Gly Gly Thr Asp Leu Phe Thr Glu Gly
                   565                 570                 575
   Leu Phe Ile Asp Tyr Arg His Phe Asp Lys Asn Gly Ile Ala Pro Arg
               580                 585                 590
   Tyr Glu Phe Gly Phe Gly Leu Ser Tyr Thr Asn Phe Thr Tyr Ser Ser
           595                 600                 605
   Leu Ser Ile Thr Ser Thr Ala Ser Ser Gly Pro Ala Ser Gly Asp Thr
       610                 615                 620
   Ile Pro Gly Gly Arg Ala Asp Leu Trp Glu Thr Val Ala Thr Val Thr
   625                 630                 635                 640
   Ala Val Val Lys Asn Thr Gly Gly Val Gln Gly Ala Glu Ala Pro Gln
                   645                 650                 655
   Leu Tyr Ile Thr Leu Pro Ser Ser Ala Pro Ser Ser Pro Pro Lys Gln
               660                 665                 670
   Leu Arg Gly Phe Ala Lys Leu Lys Leu Ala Pro Gly Glu Ser Lys Thr
           675                 680                 685
   Ala Thr Phe Ile Leu Arg Arg Arg Asp Leu Ser Tyr Trp Asp Thr Gly
       690                 695                 700
   Ser Gln Asn Trp Val Val Pro Ser Gly Ser Phe Gly Val Val Val Gly
   705                 710                 715                 720
   Ala Ser Ser Arg Asp Leu Arg Leu Asn Gly Lys Phe Asp Val Tyr
                   725                 730                 735
<210> 10
   <211> 2208
   <212> DNA
   <213> Neurospora crassa
<400> 10
   atgcaccttc gaatatttgc ggtgttggcc gcgacttccc tcgcctgggc cgagactagc      60
   gagaaacaag ctcgtcaagc tggctcaggt tttgcggcgt gggacgcagc ctattctcag     120
   gcaagcactg ctctctccaa gctttcacag caagacaagg tcaacatcgt caccggagtc     180
   ggctggaata agggcccatg tgttggcaac accccagcta ttgcatcaat cggttatccc     240
   cagctctgtt tacaagacgg ccctctcggc attcggtttg gaggaagtgt caccgcgttc     300
   acgcctggta tccaggcggc ttcaacatgg gacgtcgaac tgattcgaca gcgcggcgtc     360
   tacctcggtg cagaagccag aggggttggc gtacatgtcc ttcttggacc cgtggccgga     420
   gcgcttggca agatccccaa tggtggacgt aactgggagg gctttggtcc ggatccctac     480
   ctcacaggta ttgccatgag cgaaacaatt gaagggatcc agagcaatgg tgtacaagct     540
   tgcgccaagc acttcattct caacgaacag gagacaaacc gcgatactat cagcagtgtc     600
   gtcgacgacc gcaccatgca tgaactatac ctcttccctt ttgccgatgc cgtacactca     660
   aatgttgcaa gtgtgatgtg cagctacaac aaggtcaacg gtacgtgggc atgtgagaat     720
   gacaaaatcc agaatggcct tctcaagaaa gagctaggct tcaaaggata tgtcatgagt     780
   gattggaacg cccagcacac cacgaacggc gctgcaaaca gtggtatgga tatgacgatg 840
   ccaggcagtg actttaatgg caagacgatc ctgtggggac cacagctcaa caccgccgtc 900
   aacaatggcc aggtctccaa agcaagactg gacgacatgg ccaagcgcat tctcgcatcg 960
   tggtatttac tcgagcaaaa ctcaggctac cctgcgacta acctcaaggc caatgttcaa 1020
   ggaaaccaca aggagaacgt tcgcgcagtg gcaagagacg gcattgttct gctgaagaac 1080
   gacgataaca tcctcccgct caagaagcct agcaagctgg caatcattgg gtcatcgtcc 1140
   gttgtcaacc ctgcgggaag gaacgcctgc accgatcgag gatgcaacac cggtgcgctc 1200
   ggcatgggtt ggggctccgg cacggccgat tacccctact tcgtagcacc ctatgatgct 1260
   ctcaagacgc gggctcagtc cgacggaaca actgtcaacc tactcagctc tgacagcacc 1320
   agcggcgtag ccaacgctgc ctccggagcc gacgcggcac tagtcttcat cacagccgat 1380
   tccggcgaag gctacatcac ggtcgagggc gtgaccggcg accgtcccaa cctcgatccc 1440
   tggcacaacg gcaaccagct agtccaagcc gtggctcaag ccaacaagaa caccattgtc 1500
   gtcgtccaca gtaccggccc catcattctg gagactatcc tcgcgcagcc gggcgtcaag 1560
   gcggtcgtgt gggccggtct ccccagccaa gagaacggca acgcccttgt cgatgtccta 1620
   tacggcttgg tctctccctc gggtaagctg ccgtatacta tcgccaagag cgaaagcgac 1680
   tacggcactg ccgtgcaaag gggagggacg gatctgttca ctgagggtct gttcatcgat 1740
   taccgccact ttgacaagaa cggtatcgct ccccggtatg agttcggttt cggtctttcc 1800
   tacacgaact tcacctactc ctccctctcc atcacctcca ccgcctcctc cggtcccgcc 1860
   tcgggtgaca ccatccctgg cggccgcgcc gacctctggg aaaccgtggc aaccgtcact 1920
   gccgtcgtca aaaacacggg tggtgtgcag ggcgccgagg caccccagct atacatcacc 1980
   ttgccctctt ccgcgccgtc gagcccgccg aaacagctca gagggtttgc aaagctgaag 2040
   ctggcgcccg gggagagcaa gacagctacg ttcattttgc ggaggaggga tttgagttat 2100
   tgggatacgg gcagccagaa ttgggtggtg cctagtggca gctttggggt ggtagtgggt 2160
   gctagttcga gggatttgag gttgaatggg aagtttgatg tttattga 2208
<210> 11
   <211> 744
   <212> PRT
   <213> Trichoderma reesei
<400> 11
<210> 12
   <211> 2235
   <212> DNA
   <213> Trichoderma reesei
<400> 12
   atgcgttacc gaacagcagc tgcgctggca cttgccactg ggccctttgc tagggcagac 60
   agtcactcaa catcgggggc ctcggctgag gcagttgtac ctcctgcagg gactccatgg 120
   ggaaccgcgt acgacaaggc gaaggccgca ttggcaaagc tcaatctcca agataaggtc 180
   ggcatcgtga gcggtgtcgg ctggaacggc ggtccttgcg ttggaaacac atctccggcc 240
   tccaagatca gctatccatc gctatgcctt caagacggac ccctcggtat ccgattcggc 300
   acaggcagca cagcctttac gccgggcgtt caagcggcct cgacgtggga tgtcaatttg 360
   atccgcgaac gtggacagtt catcggtgag gaggtgaagg cctcggggat tcatgtcata 420
   cttggtcctg tggctgggcc gctgggaaag atcccgcacg gcggtcgcaa ctgggagggc 480
   ttcggtgtcg atccatatct cacgggcatt gccatggccg agacaatcga gggcctgcag 540
   tcggccggcg tgcaggcgtg cgcgaagcac tatatcctca acgagcagga gctcaatcga 600
   gaaaccattt cgagcaaccc agatgaccga actctccatg agctgtatac ttggccattt 660
   gccgacgcgg ttcacgccaa tgtcgcttct gtcatgtgct cgtacaacaa gatcaatggc 720
   agctgggcct gcgaggatca gtacacgctg cagactgtgc tgaaagacca gctggggttc 780
   ccaggctatg tcatgacgga ctggaacgca cagcacacga ctgtccaaag cgcgaattct 840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac    1440
   gcgggcgatc gcaacaacct ggatccgtgg cacaacggca atgccctggt ccaggcggtg    1500
   gccggtgcca acagcaacgt cattgttgtt gtccactccg ttggcgccat cattctggag    1560
   cagattcttg ctcttccgca ggtcaaggcc gttgtctggg cgggtcttcc ttctcaggag    1620
   agcggcaatg cgctcgtcga cgtgctgtgg ggagatgtca gcccttctgg caagctggtg    1680
   tacaccattg cgaagagccc caatgactat aacactcgca tcgtttccgg cggcagtgac    1740
   agcttcagcg agggactgtt catcgactat aagcacttcg acgacgccaa tatcacgccg    1800
   cggtacgagt tcggctatgg actgtcttac accaagttca actactcacg cctctccgtc    1860
   ttgtcgaccg ccaagtctgg tcctgcgact ggggccgttg tgccgggagg cccgagtgat    1920
   ctgttccaga atgtcgcgac agtcaccgtt gacatcgcaa actctggcca agtgactggt    1980
   gccgaggtag cccagctgta catcacctac ccatcttcag cacccaggac ccctccgaag    2040
   cagctgcgag gctttgccaa gctgaacctc acgcctggtc agagcggaac agcaacgttc    2100
   aacatccgac gacgagatct cagctactgg gacacggctt cgcagaaatg ggtggtgccg    2160
   tcggggtcgt ttggcatcag cgtgggagcg agcagccggg atatcaggct gacgagcact    2220
   ctgtcggtag cgtag                                                      2235